# EUROPÄISCHE PATENTSCHRIFT

(11) **EP 1 781 207 B1**
(45) Veröffentlichungstag und Bekanntmachung des Hinweises auf die Patenterteilung: **04.05.2011**
(21) Anmeldenummer: 05787097.4
(22) Anmeldetag: 18.08.2005
(51) Int. Cl.: A61F 2/06, A61B 17/04

(54) **VORRICHTUNG ZUM AUSSTEIFEN UND ENTLASTEN EINER GEFÄSSAUFWEITUNG**
DEVICE FOR REINFORCING AND RELIEVING A DILATED VESSEL
DISPOSITIF PERMETTANT DE RENFORCER ET DE SOULAGER UNE ZONE ELARGIE D'UN VAISSEAU

(30) Priorität: 26.08.2004 DE 102004041259
(43) Veröffentlichungstag der Anmeldung: 09.05.2007
(73) Patentinhaber: Osypka, Peter, 79618 Rheinfelden-Herten (DE)
(72) Erfinder: Osypka, Peter, 79618 Rheinfelden-Herten (DE)
(74) Vertreter: Maucher, Wolfgang
(86) Internationale Anmeldenummer: PCT/EP2005/008948
(87) Internationale Veröffentlichungsnummer: WO 2006/021365

(56) Entgegenhaltungen:
- FR-A- 2 810 876
- US-A- 5 968 053
- US-A1- 2003 120 263
- US-B1- 6 669 707

## Beschreibung

Die Erfindung betrifft eine Vorrichtung zum Aussteifen und Entlasten eines Blutgefäßes im Bereich einer Gefäßaufweitung (Aneurysma), insbesondere in einer Arterie, mit einer eine Metallarmierung aufweisenden, die Gefäßaufweitung in Gebrauchsstellung überbrückenden Gefäßstütze, die wenigstens an einem Randbereich außerhalb der Gefäßaufweitung verankerbar ist, und mit einer Zuführ-Kanüle, innerhalb welcher sich die Gefäßstütze in zusammengedrückter Position während der Einführung in das Blutgefäß befindet, wobei zum Verankern durch die Gefäßwand durchstechbare stiftartige Anker vorgesehen sind, die jeweils an einem Zugelement oder Faden befestigt und mittels eines Werkzeuges oder Stiletts durch die Gefäßwand hindurchschiebbar oder stechbar sind, und wobei das jeweilige Zugelement oder der Faden und dadurch der jeweilige Anker mit der Gefäßstütze und/oder ihrer Armierung oder mit an der Gefäßstütze oder an ihrer Armierung befindlichen Halterungen oder Ösen verbunden oder verbindbar sind.

Eine vergleichbare Vorrichtung ist aus der US 6 669 707 B1 oder aus der FR-A-2 810 876 bekannt. Dabei ist erforderlich, die Anker von der Außenseite des Gefäßes her anzubringen, d.h. die Außenseite des Gefäßes muss für die Anbringung der Verankerung zugänglich sein.

Aus der US-A-5 968 053 ist eine etwas abgewandelte Vorrichtung bekannt, bei welcher die stiftartigen Anker nicht an Zugelementen oder Fäden befestigt und mittels Werkzeug durch die Gefäßwand von innen hindurchschiebbar oder stechbar sind, sondern in diesem Falle gehen die Verankerungsstifte mit der sich in Gebrauchsstellung in ihre Ausgangslage aufweitenden Gefäßstütze mit und werden durch die Aufweitbewegung gleichzeitig durch die Gefäßwand hindurchgestochen oder in dieser angebracht, d.h. diese stiftartigen Anker müssen mit der Gefäßstütze schon vorher verbunden sein, so dass bei der Handhabung sorgfältig darauf zu achten ist, dass die Anker nicht beschädigt werden oder ihre Position verändern und vor allem auch dass eine genaue Positionierung erfolgt, da diese gleichzeitig mit der Verankerung einhergeht.

Sowohl bei einer Verankerung von der Außenseite des Gefäßes her als auch bei einer gleichzeitigen Verankerung durch die Aufweitbewegung der Gefäßstütze ist also die Sicherheit der Verankerung und der Positionierung in Frage gestellt.

Es besteht deshalb die Aufgabe, eine Vorrichtung der eingangs definierten Art zu schaffen, deren Gefäßstütze gut und sicher positionierbar und verankerbar ist.

Zur Lösung dieser Aufgabe ist die eingangs genannte Vorrichtung dadurch gekennzeichnet, dass sie in axialer Verlängerung in Vorschubrichtung vor der Gefäßstütze einen expandierbaren, für Blut durchlässigen Spreizkörper oder Käfig aufweist, der vor oder mit der Gefäßstütze aus der Zuführkanüle ausschiebbar und an der Innenseite des Gefäßes aufgrund der Expandierbarkeit oder Spreizbarkeit abstützbar ist, und dass an diesem Spreizkörper Führungen für das oder die Werkzeuge zum Verlegen der Anker von innen angeordnet sind.

Auf diese Weise ist es also möglich, zunächst die Gefäßstütze sorgfältig zu positionieren und in ihre Gebrauchsstellung zu bringen, ohne dabei gleichzeitig auch schon die Verankerung zu bewirken. Diese kann vielmehr separat von dem Positioniervorgang erfolgen. Dabei ist es auch aufgrund der Zugelemente oder Fäden an den Ankern möglich, zuerst die Anker an der Gefäßwand in einer zutreffenden Position anzubringen und danach die Gefäßstütze entlang den Fäden in ihre richtige Position zu bringen und anschließend mit den Fäden zu verbinden. Der Verankerungs- und der Positioniervorgang sind also durch die erfindungsgemäße Anordnung und Ausgestaltung der Vorrichtung voneinander unabhängig beziehungsweise "entkoppelt".

Mit Hilfe des Spreizkörpers oder Käfigs ist es möglich, während des Verankerungsvorganges die gesamte Vorrichtung innerhalb des Gefäßes festzulegen, nämlich mit dem Käfig oder Spreizkörper, der nach dem Verlassen der Zuführkanüle oder dem Zuführkatheter selbsttätig seinen Querschnitt so vergrößert, dass er sich innenseitig an das Gefäß anlegt, wodurch die von ihm getragenen Führungen für den Verankerungsvorgang gleichzeitig ihre Gebrauchsstellung erlangen. Dadurch kann vermieden werden, dass die beim Verankern auftretenden Kräfte zu einer Verschiebung der Führungen für die anderen Anker führen, d.h. die Präzision der Verankerung und der Positionierung wird vor allem durch diesen expandierbaren oder spreizbaren Körper oder Käfig erleichtert oder verbessert.

Dabei ist als besonders vorteilhaft anzusehen, dass die an den stiftartigen Ankern vorgesehenen Fäden nach dem Anbringen der Anker in der Gefäßwand als Führungshilfe für die zu positionierende Gefäßstütze dienen können, so dass durch die Anker auch die Lage der Gefäßstütze weitgehend vor ihrer eigentlichen Aufweitung und Festlegung vorherbestimmt werden kann.

Ferner können die Anker vollständig durch die Gefäßwand hindurchbewegbar sein, so dass diese in Gebrauchsstellung nur von dem Zugelement oder Faden durchsetzt wird, was eine erheblich bessere Verankerung ergibt als ein selbst in der Gefäßwand steckender Stift oder Haken.

Dabei ist es besonders günstig, wenn die Gefäßstütze relativ zu den Zugelementen oder Fäden verschiebbar mit diesen verbunden ist. Dadurch erhalten die Zugelemente eine Doppelfunktion, indem sie einerseits dazu dienen, nach dem Fixieren der stiftartigen Anker als Führung für die Gefäßstütze zu dienen, wonach sie außerdem dann beispielsweise durch Verknoten mit entsprechenden Stellen der Gefäßstütze auch deren Festlegung in der gewünschten Position ermöglichen.

Der jeweilige Anker kann als Angriffsstelle für das Werkzeug oder Stilett eine Verformung haben, die mit dem Werkzeug lösbar kuppelbar ist. Somit kann der jeweilige Anker mit Hilfe dieses Werkzeugs lösbar erfasst und in seine Gebrauchsstellung verschoben werden.

Dabei ist es zweckmäßig, wenn der Anker an seiner in Einführrichtung rückwärtigen Seite eine Querschnittsverminderung für ein aufsteckbares Ende des Werkzeugs und/oder eine am rückwärtigen Ende offene und am entgegengesetzten Ende geschlossene Aufnahmeöffnung für das in diese Öffnung passende stabförmige Werkzeug oder Stilett hat, womit der Anker in oder durch die Gefäßwand hindurchstechbar ist. Besonders günstig ist ein stift- oder stabförmiger Anker mit einer zum rückwärtigen Ende hin offenen und zu seiner Spitze geschlossenen Öffnung, in die das Ende eines Werkzeugs oder Stiletts passt, so dass es den Anker gut halten und in Stech- oder Steckrichtung entsprechend kraft- und formschlüssig beaufschlagen kann. Dennoch ist nach dem Verankern das Zurückziehen des Werkzeugs entsprechend einfach. Dabei ist der Anker zweckmäßigerweise vollständig durch die Gefäßwand hindurchstechbar, so dass nur das mit ihm verbundene Zugelement von dem Anker zurück in das Innere des Gefäßes verläuft und entsprechend gut verankert ist.

Besonders zweckmäßig für eine gute Verankerung ist es dabei, wenn das Zugelement zwischen den beiden Enden des Ankers an diesem angreift. Dadurch wird ermöglicht, dass nach dem Durchstechen des Ankers dieser sich mit seiner Längsseite an der Außenseite des Gefäßes anlegt und dass Zugelement oder der Faden etwa von der Mitte des Ankers aus zurück in das Blutgefäß verläuft. Eine solche Befestigung des Fadens oder Zugelements zwischen den Enden des Ankers, bevorzugt etwa in der Mitte zwischen beiden Enden des Ankers, erleichtert also, dass sich der Anker selbst nach dem Erreichen seiner Gebrauchsstellung flächig an die Außenseite des Gefäßes anlegt.

Das Anbringen der Anker in ihrer Gebrauchsstellung kann erheblich erleichtert werden, wenn die Führung einen Innenkanal hat, in welchem das Werkzeug oder Stilett verschiebbar ist, und wenn der Innenquerschnitt dieser Führung zumindest an ihrem äußeren Ende insbesondere etwa dem Außenquerschnitt des Ankers entspricht und den Anker in Ausgangsstellung zumindest teilweise in sich aufnimmt . Der Anker kann also am Ende der Führung ein- oder gegebenenfalls auch aufgesteckt sein, durch die das Werkzeug oder Stilett vorgeschoben wird, so dass dieses Werkzeug automatisch den Anker erfassen und dann weiterbewegen kann. Dabei können also die Anker jeweils an entsprechenden Führungen lösbar eingesteckt sein oder gegebenenfalls auch aufgesteckt sein, um so zusammen mit der Gefäßstütze in ihre Gebrauchsstellung verbracht zu werden, wo sie dann mit Hilfe des Stiletts aus der Führung herausgedrückt und durch die Gefäßwand hindurchgestochen werden, was durch eine Spitze am Ende des jeweiligen Ankers erleichtert werden kann.

Dabei kann die Vorrichtung so viele Führungen aufweisen, wie Anker vorgesehen sind. Der Benutzer kann dann nacheinander die von den einzelnen Führungen lösbar gehaltenen Anker mit Hilfe eines Werkzeugs oder Stiletts erfassen und in ihre Ankerposition bringen, wobei er das Werkzeug nacheinander in die einzelnen Führungen einführen und in ihnen so lange vorschieben muss, bis er das Ende der Führung und damit den jeweiligen stiftartige Anker erreicht hat, der dadurch automatisch von dem Werkzeug erfasst und bei einer weiteren Bewegung durch die Gefäßwand hindurchbewegt wird.

Der expandierbare Körper kann etwa parallel zueinander in axialer Richtung verlaufende Stäbe haben, die an ihren Endbereichen, insbesondere konisch oder schräg, aufeinander zulaufen, und in dem der Gefäßstütze zugewandten Endbereich des Käfigs können an den schrägen Stäben die Führungen für das Werkzeug und die Anker etwa in Richtung dieser Stäbe verlaufend angeordnet sein und am oder vor dem Übergang dieser schrägen Bereiche der Stäbe in die parallelen Verläufe enden.

Ein solcher spreizbarer Körper aus parallelen Stäben kann sich gut an der Innenseite eines Gefäßes abstützen und andrücken lassen und ist gleichzeitig für den Blutfluss durchlässig. Dabei kann in vorteilhafter Weise der Übergang dieser parallelen Stäbe zu einer mittleren Ausgangsstelle in Form von entsprechenden schrägen Verläufen dieser Stäbe ausgenutzt werden, um die Führungen für das Werkzeug und die Anker in einer günstigen Schräglage zu halten, die dann die entsprechende Richtung beim Einschieben des Werkzeugs in den jeweiligen Anker und dessen Verschieberichtung beim Durchstechen der Gefäßwand vorgibt. Dabei können in zweckmäßiger Weise die Führungen im Bereich der Umbiegung des schrägen in den axialen Bereich der einzigen Käfigstäbe münden, so dass die Anker unbehelligt von dem Körper oder Käfig, der sie zunächst mitträgt, gehandhabt werden können.

Die Fäden der Anker können an der Außenseite der Führungen insbesondere lose angeordnet sein und zu der Gefäßstütze verlaufen und die Gefäßstütze kann über die Fäden in ihre Gebrauchsstellung verschiebbar und aus der Zurührkanüle des Katheters ausschiebbar und dadurch aufweitbar sein, wobei ihre Enden beidseits der Gefäßerweiterung an die Innenseite der Gefäßwand andrückbar sind. Die Gefäßwand ist gegen Rückstellkräfte im Inneren der Zuführkanüle zunächst gehalten. Verlässt sie die Zuführkanüle weitet sie sich also selbsttätig auf, so dass der Benutzer nur dafür sorgen muss, dass sich die Gefäßstütze beim Verlassen der Kanüle in ihrer Gebrauchsstellung befindet. Beispielsweise kann er die Gefäßstütze mit Hilfe der Zuführkanüle schon in die richtige Position bringen und sie dort zunächst vom Inneren der Kanüle aus halten und die Kanüle zurückziehen. Es ist aber auch möglich, die Gefäßstütze aus der gegenüber der Gefäßaufweitung zurückgezogenen Position der Kanüle auszuschieben und zwar entlang den Fäden oder Zugelementen der Anker, bis die entsprechenden Teile oder Bereiche der Gefäßstütze, an denen die Zugelemente oder Fäden zunächst nur verschiebbar angreifen, die Anker erreicht haben, wo die Zugelemente nach der endgültigen Positionierung und Aufweitung befestigt, vorzugsweise verknotet werden können.

Der Innenquerschnitt der Gefäßstütze kann eine Größe haben, durch die der expandierbare Körper oder Käfig aufgrund seiner elastischen Nachgiebigkeit zurückziehbar ist. Beispielsweise kann dieser Körper oder Käfig aus Nitinol bestehen, welches eine gute Flexibilität aufweist. Ist also die Gefäßstütze in ihrer Gebrauchsstellung angelangt und mit den Zugelementen der Anker auch in axialer Richtung fest verbunden, beispielsweise verknotet, kann der expandierbare Körper mit den an ihm befindlichen Führungen zurückgezogen und mit der Zuführkanüle zusammen entfernt werden.

Vor allem bei Kombination einzelner oder mehrerer der vorbeschriebenen Merkmale und Maßnahmen ergibt sich eine Vorrichtung, mit der eine Gefäßstütze gut im Bereich eines Aneurysmas platziert und verankert werden kann, wobei eine hohe Festigkeit der jeweiligen Verankerungsstellen dadurch erzielt werden kann, dass die stiftartigen Anker vollständig durch die Gefäßwand hindurchstechbar sind und mit Zugelementen mit der Gefäßstütze verbunden sind, die sie beim Platzieren der Gefäßstütze auch führen können.

Auch die Zufuhr der gesamten Vorrichtung in dem Bereich der Gefäßaufweitung ist problemlos möglich, weil sich während dieses Vorgangs die entsprechenden wichtigen Teile, nämlich die Gefäßstütze selbst, aber auch der aufweitbare oder expandierbare Körper innerhalb einer entsprechend biegsamen oder flexiblen Zuführkanüle befinden, also nicht mit der Innenseite des Gefäßes in Berührung kommen.

Es wurde schon erwähnt, dass das Zugelement oder der Faden, mit welchem der jeweilige Anker verbunden ist, nach Erreichen der Gebrauchsstellung mit der Gefäßstütze fest verbunden werden kann, beispielsweise mit Hilfe eines Knotens. Dazu ist es zweckmäßig, wenn an der Gefäßstütze neben dem Zugelement oder Faden des Ankers ein weiterer Faden endet, mit dem der Faden des Ankers verknotbar ist. Beispielsweise kann an einer der metallische Armierungen der Gefäßstütze in einem stirnseitennahen Bereich eine Durchgangsöse für den Ankerfaden und unmittelbar dieser benachbart eine weitere Öse als Halterung für den weiteren Faden vorgesehen sein, so dass beide Fäden an der entsprechenden Armierung der Gefäßstütze unmittelbar nebeneinander verlaufen und in bekannter Weise chirurgisch verknotet werden können.

Nachstehend ist ein Ausführungsbeispiel der Erfindung anhand der Zeichnung näher beschrieben. Es zeigt in zum Teil schematisierter Darstellung:
- Fig. 1: Eine erfindungsgemäße Vorrichtung, bei welcher eine Gefäßstütze und ein in Vorschubrichtung davor befind- licher expandierbarer oder spreizbarer Körper mit Führungen für Anker noch in einer Zuführkanüle ange- ordnet sind, die in den Bereich einer Gefäßaufweitung einführbar und verschiebbar ist,
- Fig. 2: in vergrößertem Maßstab ein in Vorschubrichtung vor- deres Ende der Gefäßstütze mit Zugelementen, deren eines zu einem Anker führt, der an einer Führung end- seitig eingesteckt und von dort mit Hilfe eines Stiletts ausgeschoben und durch eine Gefäßwand hin- durchgestochen werden kann, wobei ein zweiter Faden an der Armierung der Gefäßstütze angeordnet ist, mit welchem der Anker-Faden nach der Verankerung und nach der Positionierung der Gefäßstütze verknotet werden kann,

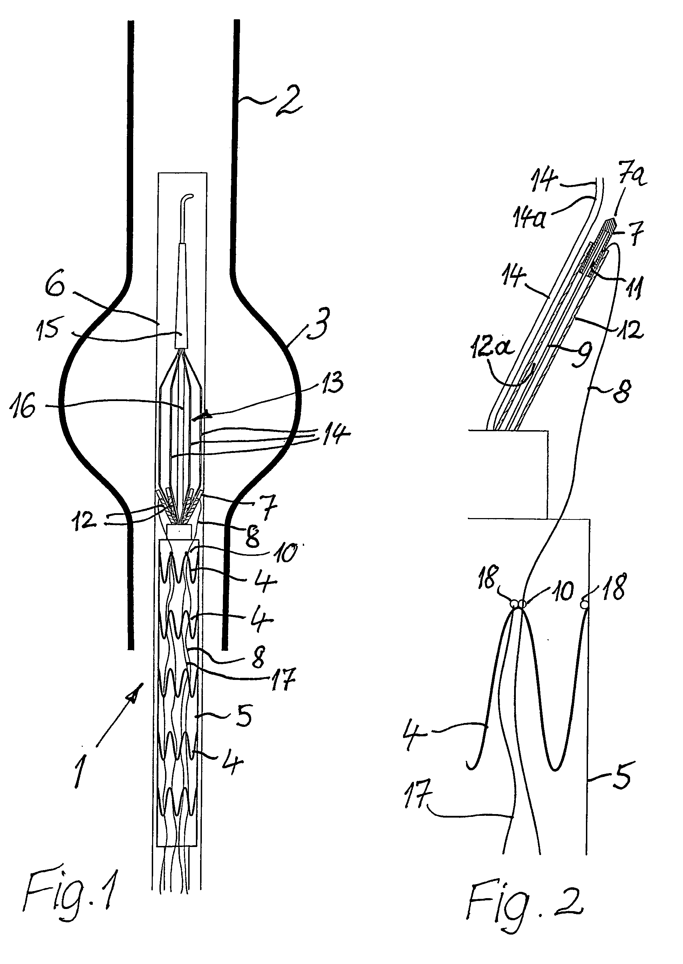

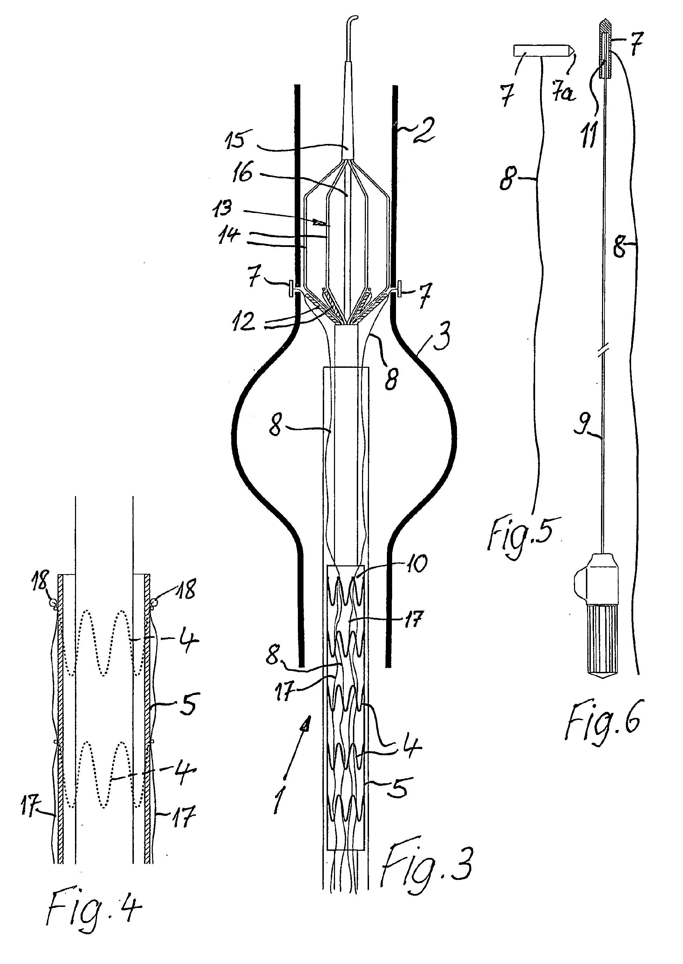

- Fig. 3: eine der Fig. 1 entsprechende Darstellung nach dem Positionieren des expandierbaren oder spreizbaren Körpers in Vorschubrichtung hinter der Gefäßerwei- terung, wodurch gleichzeitig die Führungen mit den Ankern in Ihre Gebrauchsstellung gelangt sind, wobei die Kanüle soweit zurückgezogen ist, dass sie den ex- pandierbaren Körper freigibt, die Gefäßstütze aber noch enthält und wobei die Anker bereits mit Hilfe eines Werkzeugs oder Stiletts in ihre Gebrauchsstel- lung gebracht, also durch die Gefäßwand von innen nach außen durchgestochen sind und sich relativ zu dem sie haltenden Zugelement oder Faden quergestellt haben,
- Fig. 4: in vergrößertem Maßstab einen Längsschnitt des Endes der Gefäßstütze mit ihrer wellenförmigen, am Umfang verlaufenden Armierung und an der stirnseitigen Ar- mierung befindlichen Ösen für die Zugelemente, die an den weiteren Armierungen zwischen diesen und dem Hül- senteil der Gefäßstütze verlaufen,
- Fig. 5: einen Anker mit seinem Zugelement,
- Fig. 6: den Angriff eines Werkzeugs an dem im Längsschnitt dargestellten Anker, wobei das Zugelement lose entge- gen der Einführrichtung ist, wobei das Werkzeug beim Erfassen des Ankers gemäß Fig. 2 durch eine innerhalb der Vorrichtung befindliche Führung verläuft,
- Fig. 7: eine den Fig. 1 und 3 entsprechende Darstellung nach dem Platzieren und Aufweiten der Gefäßstütze, wobei die Zuführkanüle entsprechend zurückgezogen, der spreizbare oder expandierbare Körper aber noch in ei- ner gegenüber Fig. 3 weiter vorgeschobenen Position angeordnet ist, sowie
- Fig. 8: einen Teil der Armierung der Gefäßstütze mit an dort vorgesehenen Ösen verknoteten Zugelementen zum Fest- legen der Gefäßstütze in ihrer in Fig. 7 dargestell- ten verankerten Position.

Eine im Ganzen mit 1 bezeichnete Vorrichtung dient zum Aussteifen und Entlasten eines Blutgefäßes 2 im Bereich einer Gefäßaufweitung 3 (Aneurysma), insbesondere in einer Arterie, gegebenenfalls in der Aorta.

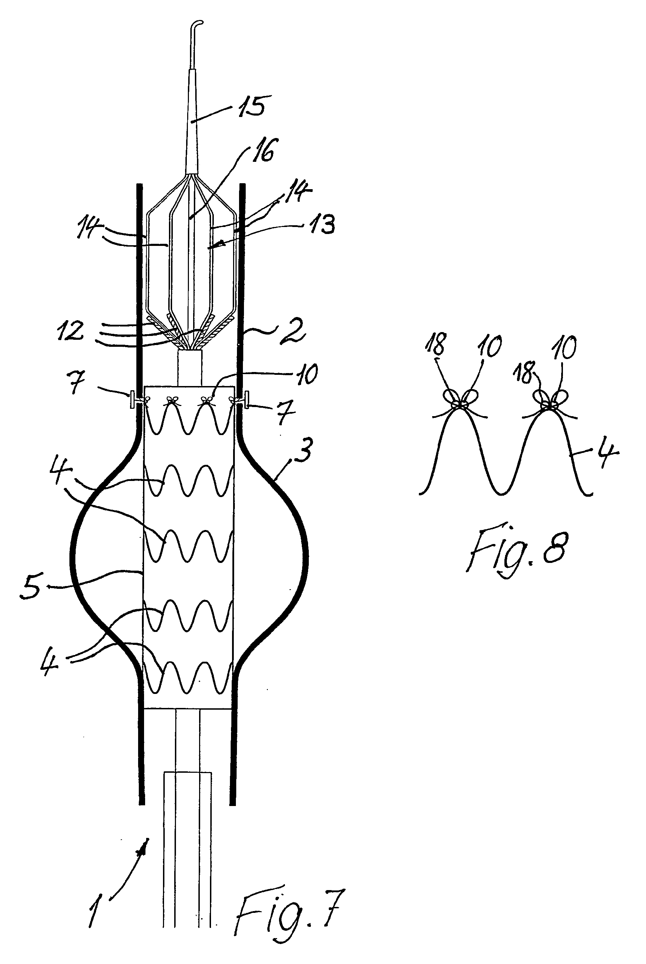

Die Vorrichtung 1 weist dazu eine mit einer Metallarmierung 4 versehene, die Gefäßaufweitung 3 in Gebrauchsstellung (Fig. 7) überbrückende Gefäßstütze 5 auf, die gemäß Fig. 7 an einem Randbereich außerhalb der Gefäßaufweitung 3 in noch zu beschreibender Weise verankerbar ist.

Zu der Vorrichtung 1 gehört auch eine Zuführkanüle 6, innerhalb welcher sich die Gefäßstütze 5 in zusammengedrückter oder gegebenenfalls auch gefalteter Position während der Einführung in das Blutgefäß 2 und durch das Blutgefäß hindurch bis in den Bereich der Gefäßaufweitung 3 befindet, das heißt innerhalb der Zuführkanüle 6 hat die Gefäßstütze 5 zunächst eine geringere radiale Ausdehnung als in Gebrauchsstellung, so dass sie mit Hilfe der Zuführkanüle 6 durch ein entsprechendes Blutgefäß 2 an die Benutzungsstelle, also die Aufweitung 3 gebracht werden kann.

Zum Verankern der Gefäßstütze 5 in ihrer Gebrauchsstellung sind von innen durch die Gefäßwand gemäß Fig. 3 und 7 durchstechbare stiftartige Anker 7 vorgesehen, die jeweils an einem Zugelement oder Faden 8 befestigt und mittels eines Werkzeugs oder Stiletts 9 (vgl. Fig. 2 und 6) durch die Gefäßwand hindurchschiebbar oder stechbar sind.

Die stiftartigen Anker 7 haben dazu an ihrem in Vorschubrichtung vorderen Ende eine Spitze 7a, die diesen Stechvorgang erleichtert.

Gemäß den Fig. 2, 7 und 8 ist das jeweilige Zugelement oder der Faden 8 und dadurch auch der jeweilige Anker 7 mit der Gefäßstütze 5 und gleichzeitig mit ihrer Armierung 4 beziehungsweise mit an der Gefäßstütze 5 oder an ihrer Armierung 4 befindlichen Halterungen oder Ösen 10 verbunden oder verbindbar.

Dabei erkennt man anhand der Fig. 1 bis 4, dass die Gefäßstütze 5 zunächst relativ zu den Zugelementen oder Fäden 8 verschiebbar mit diesen verbunden ist, das heißt die Fäden 8 verlaufen gleitend durch die an der Armierung 4 befindlichen Ösen 10, so dass nach dem Setzen der Anker 7 die Gefäßstütze 5 entlang den Fäden 8 verschoben werden kann, bis sie ihre Gebrauchsstellung erreicht hat. Die Gefäßstütze 5 ist also zumindest zunächst relativ zu den Zugelemente oder Fäden 8 verschiebbar, gleichzeitig aber doch damit schon verbunden.

Gemäß Fig. 2 und 6 hat der Anker 7 als Angriffsstelle für das Werkzeug oder Stilett 9 eine Verformung, die mit dem Werkzeug 9 beziehungsweise seinem Ende lösbar kuppelbar ist. Man erkennt, dass der Anker 7 an seinem in Einführrichtung rückwärtigen Ende eine offene und am entgegengesetzten Ende im Bereich seiner Spitze 7a geschlossene Aufnahmeöffnung 11 für das in diese Öffnung 11 passende stabförmige Werkzeug oder Stilett 9 hat, womit der Anker 7 in oder durch die Gefäßwand hindurchstechbar ist. Wird also das Ende des Stiletts 9 in die Öffnung 11 eingeschoben und dann das Stilett 9 in der gleichen Richtung weiterbewegt, nimmt es automatisch den Anker 7 mit, bis er seine Gebrauchsstellung erreicht hat. Danach kann das Stilett 9 auf einfache Weise zurückgezogen werden, wodurch der Anker in seiner Gebrauchsstellung verbleibt, zumal er sich relativ zu dem Zugelement oder Faden 8 dann querstellt, also nicht durch die Öffnung zurückgleiten kann, die er selbst in der Gefäßwand verursacht hat.

Damit diese Querstellung des Ankers 7 gegenüber dem Zugelement oder Faden 8 automatisch erfolgt, greift das Zugelement 8 zwischen den beiden Enden des Ankers 7, im Ausführungsbeispiel etwa in der Mitte, an diesem an. Diese Querstellung ist in Fig. 3 und 7 und vergrößert noch einmal in Fig. 5 erkennbar.

Damit das Werkzeug 9 die einzelnen Anker 7 - nacheinander - erfassen und treffen kann, weist die Vorrichtung 1 für jeden Anker 7 eine Führung 12 auf, die einen Innenkanal 12a hat (vgl. Fig. 2), in welchem das Werkzeug 9 oder Stilett in axialer Richtung verschiebbar ist. Dabei entspricht der Innenquerschnitt beziehungsweise der Innenkanal 12a dieser Führung 12 zumindest am äußeren Ende etwa dem Außenquerschnitt des Ankers 7, so dass der Anker 7 gemäß Fig. 2 und auch gemäß Fig. 1 teilweise in diese Führung 12 eingesteckt sein kann, und zwar etwa bis zur Mitte, wo das Zugelement beziehungsweise der Faden 8 abgeht. Dieser hängt dabei außenseitig an der Führung 12 und verläuft unmittelbar zu der Außenseite der Gefäßstütze 5 und der dort vorgesehenen Öse 10.

Man erkennt in den Fig. 1, 3 und 7, dass dabei so viele derartige Führungen 12 vorgesehen sind, wie Anker 7 zur Verankerung der Gefäßstütze 5 dienen sollen, beispielsweise vier, fünf, sechs oder acht Führungen 12 für entsprechend viele Anker 7. Es genügt aber ein Werkzeug 9, um die einzelnen Führungen 12 nacheinander zu benutzen, um die an den Enden durch Kraftschluss gehaltenen Anker 7 nacheinander zu erfassen und durch die Gefäßwand hindurchzuschieben.

Gemäß Fig. 1, 3 und 7 weist die Vorrichtung 1 in axialer Verlängerung beziehungsweise in Vorschubrichtung vor der Gefäßstütze 5 einen expandierbaren oder spreizbaren, für Blut durchlässigen Körper oder Käfig 13 auf, der zunächst in der Zuführkanüle 6 angeordnet ist (Fig. 1) und vor der Gefäßstütze 5 aus der Zuführkanüle 6 ausgeschoben werden kann. Dieser Spreizkörper 13, der aufgrund seiner käfigartigen Ausbildung für Blut durchlässig ist, stützt sich dann in Gebrauchsstellung gem. Fig. 3 aufgrund seiner selbsttätigen Expandierbarkeit oder Spreizbarkeit an der Innenseite des Gefäßes 2 ab und fixiert damit wichtige Teile der Vorrichtung 1 zumindest vorläuft. An diesem Spreizkörper 13 sind nämlich die Führungen 12 für das oder die Werkzeuge 9 zum Verlegen der Anker 7 angeordnet. Wird also dieser Körper oder Käfig 13 in die in Fig. 3 dargestellte Position gebracht und dort abgestützt, können die Anker 7 entsprechend positioniert und fixiert werden. Gleichzeitig gelangen dadurch die Fäden 8 in eine Endposition, so dass nunmehr relativ zu diesen Fäden 8 auch die Gefäßstütze 5 aus der Zuführkanüle 6 ausgeschoben werden kann, wobei gleichzeitig oder danach die Zuführkanüle 6 zurückzuziehen ist, bis die in Fig. 7 dargestellte Position erreicht ist.

Der expandierbare Körper 13 hat etwa parallel zueinander in axialer Richtung verlaufende Stäbe 14, die an ihren Enden oder Endbereichen konisch oder schräg aufeinander zulaufen, wie es besonders gut in den Fig. 3 und 7 erkennbar ist. Dabei sind die voneinander abgewandten Enden dieser schrägen Bereiche von einer zentralen Halterung 15 erfasst, von welcher ein zentraler Stab 16 zu der Zuführkanüle 6 verläuft und somit den expandierbaren Körper 13 beidseits hält und trägt.

In dem der Gefäßstütze 5 zugewandten Endbereich des Käfigs oder Körpers 13 sind an den schrägen Stäben dieses Körpers 13 die Führungen 12 für das Werkzeug 9 und die Anker 7 etwa in der schrägen Richtung dieser Stabbereiche verlaufend angeordnet, was deutlich in Fig. 2 aber auch gut in den Fig. 1, 3 und 7 erkennbar ist. Dabei enden diese Führungen 12 am oder vor dem Übergang der schrägen Bereiche der Stäbe 14 in deren parallele Verläufe. In Fig. 2 ist die Krümmung 14a des zunächst schräg verlaufenden Stabes 14 und damit der Übergang in den axialen Verlauf dieses Stabes 14 zu erkennen und man sieht, dass der Anker 7 mit seiner Spitze 7a etwa auf der Höhe diese Krümmung 14a angeordnet ist, bevor er in seine Gebrauchsstellung verschoben wird. Somit kann diese Verschiebung des Ankers 7 aus der Führung 12 heraus stattfinden, ohne durch die Stäbe 14 behindert zu werden.

Dazu trägt auch bei, dass sich die Führung 12 an der Außenseite des expandierbaren Körpers oder Käfigs 13, also in Fig. 2 unterhalb des Stabes 14 befindet.

Wie bereits erwähnt, sind die Fäden 8 der Anker 7 an der Außenseite der Führungen 12 lose angeordnet und verlaufen zu der Gefäßstütze 5 beziehungsweise zu einer an deren Armierung 4 angeordneten Öse 10. Die Gefäßstütze 5 ist also über diese Fäden 8 in ihre Gebrauchsstellung verschiebbar und aus der Zuführkanüle 6 ausschiebbar, wobei umgekehrt auch die Zuführkanüle 6 relativ zu der Gefäßstütze 5 zurückgezogen werden kann. Die selbsttätig aufweitbare Gefäßstütze 5 ist also nach dem Verlassen der Zuführkanüle 6 automatisch aufweitbar und erhält die in Fig. 7 dargestellte Form, wobei ihre Enden beidseits der Gefäßerweiterung 3 an die Innenseite der Gefäßwand andrückbar sind, ohne aber dabei eine zu große Druckkraft ausüben zu müssen, weil die eigentliche axiale Festlegung mit Hilfe der Anker 7 erfolgt.

Der Innenquerschnitt der Gefäßstütze 5 hat eine Größe, durch die der expandierbare Körper 13, auch aufgrund seiner elastischen Nachgiebigkeit, da er zum Beispiel als Nitinol bestehen kann, zurückziehbar ist. Fig. 7 zeigt den Körper 13 kurz vor seiner Zurückziehung, die ihn zusammen mit den Führungen 12 dann auch wieder in die zurückgezogene Einführkanüle 6 hineinbringen kann.

In Fig. 2, 4 und auch in Fig. 3 erkennt man, dass an der Gefäßstütze 5 neben dem jeweiligen Zugelement oder Faden 8 des Ankers 7 ein weiterer Faden 17 zweckmäßigerweise an einer der Öse 10 benachbarten weiteren Öse 18 endet und angreift, mit welchem der Faden 8 des Ankers 7 verknotbar ist. Die verknotete Anordnung ist in Fig. 7 und vergrößert in Fig. 8 dargestellt. Nach diesem Verknoten der Fäden 8 und 17 ist also die Gefäßstütze 5 endgültig festgelegt und verankert. Dabei genügt die Anbringung der Anker 7 an der einen Endseite, weil der Blutstrom so gerichtet ist, dass er zunächst in das die Anker 7 aufweisende Ende der Gefäßstütze 5 eintritt.

Die Metallarmierung 4 an der Außenseite der Gefäßstütze 5 läuft wellenförmig an deren Umfang um und ist in axialer Richtung mehrfach vorhanden. In Fig. 4 ist angedeutet, dass dabei diese Armierung 4 an der Außenseite der Gefäßstütze 5 verläuft, so dass die Fäden 8 und 17 unter der Armierung 4, das heißt zwischen dieser und der Außenseite der Gefäßstütze 5, verlaufen, also von den Armierungen 4 geführt und in radialer Richtung gehalten werden können.

Die Vorrichtung 1 zum Aussteifen und Entlasten eines Blutgefäßes 2 im Bereich einer Gefäßaufweitung 3 weist eine Gefäßstütze 5 mit Metallarmierung 4 auf, welche in Gebrauchsstellung die Gefäßaufweitung 3 überbrückt und außerhalb der Gefäßaufweitung 3 an dem Blutgefäß 2 verankerbar ist. Zu dieser Vorrichtung 1 gehört eine Zuführkanüle 6, innerhalb welcher sich die Gefäßstütze 5 während des Einführens in das Blutgefäß 2 in einer Form befindet, in welcher sie eine geringe radiale Ausdehnung hat, aus der sie nach dem Verlassen der Gefäßstütze 5 selbsttätig aufweitbar ist, um dann dem Gefäßquerschnitt etwa zu entsprechen.

Zum Verankern der Gefäßstütze 5 sind von innen durch die Gefäßwand durchstechbare stiftartige Anker 7 vorgesehen, die jeweils an einem Zugelement oder Faden 8 befestigt sind und sich nach dem Durchstechen durch die Gefäßwand querstellen. Mit Hilfe des Zugelements 8 können die Anker 7 mit der Gefäßstütze 5 nach deren Positionierung verbunden werden.

In zweckmäßiger Weise können dabei diese Ankerfäden 8 auch beim Verschieben der Gefäßstütze 5 in ihre Gebrauchsstellung deren Führung unterstützen.

## Patentansprüche

1. Vorrichtung (1) zum Aussteifen und Entlasten eines Blutgefäßes (2) im Bereich einer Gefäßaufweitung (3) mit einer eine Metallarmierung (4) aufweisenden, die Gefäßaufweitung in Gebrauchsstellung überbrückenden Gefäßstütze (5), die wenigstens an einem Randbereich außerhalb der Gefäßaufweitung (3) verankerbar ist, und mit einer Zuführkanüle (6), innerhalb welcher sich die Gefäßstütze (5) in in radialer Richtung zusammengedrückter Position während der Einführung in das Blutgefäß (2) befindet, wobei zum Verankern durch die Gefäßwand durchstechbare stiftartige Anker (7) vorgesehen sind, die jeweils an einem Zugelement oder Faden (8) befestigt und mittels eines Werkzeugs oder Stiletts (9) durch die Gefäßwand hindurchschiebbar oder stechbar sind, und wobei das jeweilige Zugelement oder der Faden (8) und **dadurch** der jeweilige Anker (7) mit der Gefäßstütze (5) und/oder ihrer Armierung (4) oder mit an der Gefäßstütze (5) oder an ihrer Armierung (4) befindlichen Halterungen oder Ösen (10) verbunden oder verbindbar sind, **dadurch gekennzeichnet, dass** sie in axialer Verlängerung in Vorschubrichtung vor der Gefäßstütze (5) einen expandierbaren, für Blut durchlässigen Körper oder Käfig (13) aufweist, der vor oder mit der Gefäßstütze (5) aus der Zuführkanüle (6) ausschiebbar und an der Innenseite des Gefäßes (2) aufgrund der Expandierbarkeit oder Spreizbarkeit abstützbar ist, und dass an diesem Spreizkörper (13) Führungen für das oder die Werkzeuge (9) zum Verlegen der Anker (7) von innen angeordnet sind.

2. Vorrichtung nach Anspruch 1, **dadurch gekennzeichnet, dass** die Gefäßstütze (5) relativ zu den Zugelementen oder Fäden (8) der Anker (7) verschiebbar und mit diesen verbunden ist.

3. Vorrichtung nach Anspruch 1 oder 2, **dadurch gekennzeichnet, dass** der Anker (7) als Angriffsstelle für das Werkzeug oder Stilett (9) eine Verformung hat, die mit dem Werkzeug (9) lösbar kuppelbar ist.

4. Vorrichtung nach einem der Ansprüche 1 bis 3, **dadurch gekennzeichnet, dass** der Anker (7) an seiner in Einführrichtung rückwärtigen Seite eine Querschnittsverminderung für das aufsteckbare Ende des Werkzeugs (9) und/oder eine am rückwärtigen Ende offene und am entgegengesetzten Ende geschlossene Aufnahmeöffnung (11) für das in dieser Öffnung (11) passende stabförmige Werkzeug oder Stilett (9) hat, womit der Anker (7) in oder durch die Gefäßwand hindurchstechbar ist.

5. Vorrichtung nach einem der Ansprüche 1 bis 4, **dadurch gekennzeichnet, dass** das Zugelement (8) zwischen den beiden Enden des Ankers (7), insbesondere etwa in der Mitte, an diesem angreift.

6. Vorrichtung nach einem der Ansprüche 1 bis 5, **dadurch gekennzeichnet, dass** die Führung (12) für das stabförmige Werkzeug (9) einen Innenkanal (12a) hat, in welchem das Werkzeug (9) oder Stilett verschiebbar ist, und dass der Innenquerschnitt dieser Führung (12) zumindest an ihrem äußeren Ende insbesondere etwa dem Außenquerschnitt des Ankers (7) entspricht und den Anker in sich zumindest teilweise aufnimmt.

7. Vorrichtung nach einem der Ansprüche 1 bis 6, **dadurch gekennzeichnet, dass** sie so viele Führungen (12) aufweist, wie Anker vorgesehen (7) sind.

8. Vorrichtung nach einem der Ansprüche 1 bis 7, **dadurch gekennzeichnet, dass** der expandierbare Körper (13) etwa parallel zueinander in axialer Richtung verlaufende Stäbe (14) hat, die an ihren Endbereichen, insbesondere konisch oder schräg, aufeinander zulaufen, und dass in dem der Gefäßstütze (5) zugewandten Endbereich des Käfigs (13) an den schrägen Stäben die Führungen (12) für das Werkzeug (9) und die Anker (7) etwa in Richtung dieser Stäbe verlaufend angeordnet sind und am oder vor dem Übergang dieser schrägen Bereiche der Stäbe (14) in die parallelen Verläufe enden.

9. Vorrichtung nach einem der Ansprüche 1 bis 8, **dadurch gekennzeichnet, dass** die Fäden (8) der Anker (7) an der Außenseite der Führungen (12) insbesondere lose angeordnet sind und zu der Gefäßstütze (5) verlaufen und dass die Gefäßstütze (5) über die Fäden (8) in ihre Gebrauchsstellung verschiebbar und aus der Zuführkanüle (6) ausschiebbar und **dadurch** aufweitbar ist, wobei ihre Enden beidseits der Gefäßerweiterung (3) an die Innenseite der Gefäßwand andrückbar sind.

10. Vorrichtung nach einem der Ansprüche 1 bis 9, **dadurch gekennzeichnet, dass** der Innenquerschnitt der Gefäßstütze (5) in Gebrauchsstellung eine Größe hat, durch die der expandierbare Körper (13) aufgrund seiner elastischen Nachgiebigkeit, beispielsweise Nitinol, zurückziehbar ist.

11. Vorrichtung nach einem der Ansprüche 1 bis 10, **dadurch gekennzeichnet, dass** an der Gefäßstütze (5) neben dem Zugelement oder Faden (8) des Ankers (7) ein weiterer Faden (17) endet, mit dem der Faden (8) des Ankers (7) verknotbar ist.

## Claims

1. Device (1) for reinforcing and relieving a blood vessel (2) in the region of a dilated vessel section (3), with a vessel support (5) that comprises a metal reinforcement (4) and bridges the dilated vessel section in the position of use, said support being adapted to be anchored at least in one edge region outside the dilated vessel section (3), and with a supply cannula (6) inside which the vessel support (5) is located in a radially compressed position during insertion into the blood vessel (2), while for anchoring purposes pin-like anchors (7) that can be stuck through the vessel wall are provided, each anchor being secured to one tensioning element or filament (8) and being adapted to be pushed or stuck through the vessel wall using a tool or stiletto (9), and wherein the respective tensioning element or the filament (8) and hence the respective anchor (7) is or may be connected to the vessel support (5) and/or its reinforcement (4) or holders or eyelets (10) located on the vessel support (5) or on its reinforcement (4), **characterised in that** it comprises, on an axial extension in front of the vessel support (5) in the direction of advance, an expandable body or cage (13) that is pervious to blood, that can be pushed out of the supply cannula in front of or with the vessel support (5) and can be supported on the inside of the vessel (2) due to its expandability or spreadability, and **in that** guides for the tool or tools (9) are arranged on this spreading body (13), for moving the anchors (7) from inside.

2. Device according to claim 1, **characterised in that** the vessel support (5) is movable relative to the tensioning elements or filaments (8) of the anchors (7) and is connected thereto.

3. Device according to claim 1 or 2, **characterised in that** the anchor (7) has, as a point of engagement for the tool or stiletto (9), a deformation that can be releasably coupled to the tool (9).

4. Device according to one of claims 1 to 3, **characterised in that** the anchor (7) has on its side that is rearmost in the direction of insertion a reduction in cross-section for the insertable end of the tool (9) and/or a receiving opening (11) open at the rearward end and closed at the opposite end for the rod-shaped tool or stiletto (9) that fits in this opening (11), by means of which the anchor (7) can be pushed into or through the vessel wall.

5. Device according to one of claims 1 to 4, **characterised in that** the tensioning element (8) acts on the anchor (7) between the two ends thereof, more particularly substantially in the middle thereof.

6. Device according to one of claims 1 to 5, **characterised in that** the guide (12) for the rod-shaped tool (9) has an inner channel (12a) in which the tool (9) or stiletto can be moved, and **in that** the internal cross-section of this guide (12) substantially corresponds in particular, at least at its outer end, to the external cross-section of the anchor (7), and at least partially accommodates the anchor within itself.

7. Device according to one of claims 1 to 6, **characterised in that** it has as many guides (12) as there are anchors (7).

8. Device according to one of claims 1 to 7, **characterised in that** the expandable body (13) has rods (14) extending substantially parallel to one another in the axial direction, which converge with one another, particularly conically or diagonally, at their end regions, and **in that** in the end region of the cage (13) facing the vessel support (5), on the diagonal rods, are provided the guides (12) for the tool (9) and the anchors (7) extending substantially in the direction of these rods and terminating at or before the transition of these diagonal regions of the rods (14) into the parallel portions.

9. Device according to one of claims 1 to 8, **characterised in that** the filaments (8) of the anchors (7) are arranged loosely, in particular, on the outside of the guides (12) and extend towards the vessel support (5), and **in that** the vessel support (5) can be pushed over the filaments (8) into its position of use and out of the supply cannula (6) and in this way can be expanded, its ends on either side of the dilated vessel section (3) being adapted to be pressed against the inside of the vessel wall.

10. Device according to one of claims 1 to 9, **characterised in that** the internal cross-section of the vessel support (5) in the position of use is of a size through which the expandable body (13) can be retracted thanks to its elastic flexibility, being of Nitinol, for example.

11. Device according to one of claims 1 to 10, **characterised in that** besides the tensioning element or filaments (8) of the anchor (7) there is another filament (17) terminating on the vessel support (5), to which the filament (8) of the anchor (7) can be tied.

## Revendications

1. Dispositif (1) pour renforcer et soulager un vaisseau sanguin (2) au niveau d'une dilatation de vaisseau (3) avec un stent (5) présentant une armature métallique (4), qui ponte la dilatation de vaisseau en position d'utilisation et peut être ancré au moins à une zone de bord à l'extérieur de la dilatation de vaisseau (3), et avec une canule d'insertion (6) à l'intérieur de laquelle se trouve le stent (5) dans une position comprimée en direction radiale pendant son introduction dans le vaisseau sanguin (2), des ancres (7) en forme de broche capables de percer la paroi de vaisseau de l'intérieur étant prévues pour l'ancrage, qui sont chacune fixées à un élément de traction ou un fil (8) et peuvent être poussées ou enfoncées à travers la paroi de vaisseau au moyen d'un outil ou stylet (9), et l'élément de traction ou le fil (8) respectif et de ce fait l'ancre (7) respective étant reliés ou pouvant être reliés au stent (5) et/ou à son armature (4) ou à des moyens de fixation ou des oeillets (10) se trouvant sur le stent (5) ou sur son armature (4), **caractérisé en ce qu'**il présente en prolongement axial, avant le stent (5) dans la direction d'avancement, un corps ou une cage (13) extensible, perméable au sang, qui peut être sorti(e) par coulissement de la canule d'insertion (6) avant ou avec le stent (5) et appuyé contre le côté intérieur du vaisseau (2) en raison de son extensibilité ou expansibilité, et que des guides pour le ou les outils (9) de pose des ancres (7) depuis l'intérieur sont disposés sur ce corps expansible (13).

2. Dispositif selon la revendication 1, **caractérisé en ce que** le stent (5) peut coulisser par rapport aux éléments de traction ou fils (8) des ancres (7) et est relié à ceux-ci.

3. Dispositif selon la revendication 1 ou 2, **caractérisé en ce que** l'ancre (7) possède en tant que point d'attaque pour l'outil ou le stylet (9) une déformation qui peut être accouplée de manière détachable avec l'outil (9).

4. Dispositif selon une des revendications 1 à 3, **caractérisé en ce que** l'ancre (7) possède sur son côté arrière dans la direction d'introduction une diminution de section pour l'extrémité enfichable de l'outil (9) et/ou une ouverture de réception (11) ouverte à l'extrémité arrière et fermée à l'extrémité opposée pour l'outil ou le stylet (9) en forme de tige adapté à cette ouverture (11), permettant à l'ancre (7) d'être enfoncée dans ou à travers la paroi de vaisseau.

5. Dispositif selon une des revendications 1 à 4, **caractérisé en ce que** l'élément de traction (8) agit entre sur l'ancre (7) entre les deux extrémités de celle-ci, en particulier à peu près en son centre.

6. Dispositif selon une des revendications 1 à 5, **caractérisé en ce que** le guide (12) pour l'outil (9) en forme de tige possède un canal intérieur (12a) dans lequel l'outil (9) ou le stylet peuvent coulisser, et que la section intérieure de ce guide (12) correspond, au moins à son extrémité extérieure, en particulier à peu près à la section extérieure de l'ancre (7) et loge l'ancre au moins partiellement.

7. Dispositif selon une des revendications 1 à 6, **caractérisé en ce qu'**il est prévu autant de guides (12) que d'ancres (7).

8. Dispositif selon une des revendications 1 à 7, **caractérisé en ce que** le corps extensible (13) possède des tiges (14) s'étendant à peu près parallèlement les unes aux autres en direction axiale, qui convergent, en particulier de manière conique ou oblique, dans leurs parties terminales, et que les guides (12) pour l'outil (9) et les ancres (7) sont disposés sur les tiges obliques dans la partie terminale de la cage (13) tournée vers le stent (5), s'étendent à peu près dans la direction de ces tiges et se terminent au niveau de ou avant le passage de ces zones obliques aux extensions parallèles de ces tiges (14).

9. Dispositif selon une des revendications 1 à 8, **caractérisé en ce que** les fils (8) des ancres (7) sont disposées, en particulier de manière lâche, sur le côté extérieur des guides (12) et s'étendent vers le stent (5), et que l'on peut faire coulisser le stent (5) dans sa position d'utilisation au moyen des fils (8) et le faire sortir de la canule d'insertion (6) et ainsi permettre son élargissement, ses extrémités pouvant être appuyées contre le côté intérieur de la paroi de vaisseau des deux côtés de la dilatation de vaisseau (3).

10. Dispositif selon une des revendications 1 à 9, **caractérisé en ce que** la section intérieure du stent (5) en position d'utilisation a une grandeur qui permet de retirer le corps extensible (13), par exemple en nitinol, du fait de sa souplesse élastique.

11. Dispositif selon une des revendications 1 à 10, **caractérisé en ce qu'**un autre fil (17), auquel le fil (8) de l'ancre (7) peut être noué, se termine au niveau du stent (5) à côté de l'élément de traction ou du fil (8) de l'ancre (7).
